# EUROPEAN PATENT SPECIFICATION

(11) **EP 1 756 226 B1**
(45) Date of publication and mention of the grant of the patent: **09.09.2009**
(21) Application number: 05736574.4
(22) Date of filing: 19.04.2005
(51) Int. Cl.: C08L 83/12, C08J 3/205

(54) **SILICONE VESICLES**
SILIKONVESIKEL
VESICULES DE SILICONE

(30) Priority: 20.04.2004 US 563663 P
(43) Date of publication of application: 28.02.2007
(73) Proprietor: Dow Corning Corporation, Midland, MI 48686-0994 (US)
(72) Inventor: LIN, Shaow, Midland, MI 48642 (US)
(74) Representative: Polypatent
(86) International application number: PCT/US2005/013289
(87) International publication number: WO 2005/103157

(56) References cited:
- EP-A- 0 483 465
- EP-A- 0 724 876
- US-A- 5 958 433
- US-A1- 2003 220 425

## Description

### FIELD OF THE INVENTION

The present invention provides a process for preparing silicone vesicles by combining and mixing an organopolysiloxane having at least one hydrophilic substituent group, a water miscible volatile solvent, and water. The invention also relates to the vesicle compositions produced by the method and their use in various personal, household, and health care applications.

### BACKGROUND OF THE INVENTION

Silicone surfactants have been designed for various applications by combining a hydrophobic organopolysiloxane with various hydrophilic moieties. For example, the silicone surfactants known as silicone polyethers are based on copolymer structures of polydimethylsiloxane having pendant polyoxyalkylene groups. Such materials find wide utility in many personal, household, and health care compositions as emulsifiers, wetting agents, and general-purpose aqueous surfactants.

The aggregation behavior of silicone polyethers has been discussed along with their ability to form vesicles. For example, US Patents 5,364,633 and 5,411,744 by Hill teaches the self-assembly of silicone vesicles in aqueous dispersions of certain silicone polyethers. PCT application US03/38455 by Lin teaches the entrapment of various oils in silicone vesicles and their use in various personal care formulations. US-A-5,958,433 discloses a dispersion of a water-immiscible phase, in an external aqueous phase by means of lipid vesicles comprising at least one silicone surfactant, for the treatment of the skin, the mucous membranes, the nails, the scalp and the hair and in particular, greasy skin.

While these references represent advancements in the art, a need still exists to create silicone vesicles having improved stability. For example, the silicone vesicles formed from the self-assembly procedures can lack durability. This lack of durability can limit the processing condition they can be subject to in the formation of various finished products.
Furthermore, such self-assembled silicone vesicles can lack the ability to provide sustained release of loaded actives over an extended period of time. Thus, a need exists for a process to prepare silicone vesicles having improved durability and the ability to release loaded actives over an extended period of time.

The present inventors have discovered a process to prepare silicone vesicles having improved durability and the ability to release loaded actives over an extended period of time.

### SUMMARY OF THE INVENTION

The present invention provides a process for preparing a vesicle composition according to claim 1.

The present invention also relates to the vesicle compositions obtained from the inventive process, and the vesicle compositions further comprising a personal, household, health care ingredient.

The present invention further relates to personal, household, health care product compositions containing the vesicle compositions.

### DETAILED DESCRIPTION OF THE INVENTION

The first step in the process of the present invention involves combining;
A) an organopolysiloxane having at least one hydrophilic substituent group,
B) a water miscible volatile solvent,
with water to form an aqueous dispersion.

Component A) is an organopolysiloxane having at least one hydrophilic substituent group selected from silicone polyethers as defined below Component (A) is a silicone polyether and has a structure represented by: 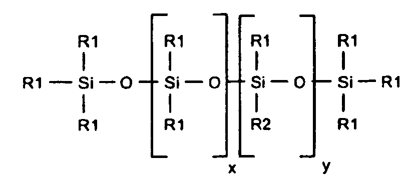 or 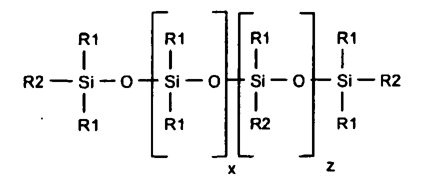 A cyclic polyether of the type shown below can also be used. 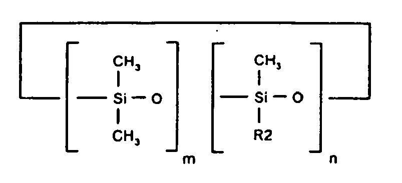 In these structures, R1 represents an alkyl group containing 1-6 carbon atoms such as methyl, ethyl, propyl, butyl, pentyl, and hexyl; R2 represents the group - (CH₂)ₐO(C₂H₄O)_{b}(C₃H₆O)_{c}R3; x has a value of 1-1,000, alternatively 1 - 500, or alternatively 10 - 250; y has a value of 1-500, alternatively 1- 100, or alternatively 2 - 50; z has a value of 1-500, or alternatively 1 - 100; m has a value of 3-5; n is one; a has a value of 3-6; b has a value of 4-20; c has a value of 0-5; and R3 is hydrogen, a methyl group, or an acyl group such as acetyl. Typically, R1 is methyl; b is 6-12; c is zero; and R3 is hydrogen.

The silicone surfactants can be prepared by any of the techniques known in the art, and many are commercially available. For example, rake type SPEs are derived most commonly from hydrosilylating SiH sites of MDₓD'_{y}M with allyloxy-functional polyethers in the presence of Pt catalyst.

The process of the present invention is particularly useful to prepare silicone vesicle compositions containing a silicone polyether having silicone/polyether molar ratios such that the silicone polyether aqueous solublility varies from those having only slightly water solublility or dispersiblility to insoluble ones. This silicone/polyether ratio can be represented as value of x/y or x/z in the silicone polyether structures shown above and ranges from 5/1 to 50/1, alternatively from 10/1 to 50/1, or alternatively from 20/1 to 50/1. Such silicone polyethers are prepared from a SiH functional organopolysiloxane having a D/D' ratio ranging from 5/1 to 50/1, alternatively from 10/1 to 50/1 or alternatively from 20/1 to 50/1, where D' represents a SiH disiloxy unit (for example MeHSiO).

Component B) is a water-miscible volatile solvent. As used herein "water-miscible" means the solvent forms a dispersion with water at room temperature for at least several hours. "Volatile" means the solvent has a higher vapor pressure than water at various temperatures. As such, when the aqueous dispersion of the organopolysiloxane and solvent are subjected to conditions to remove the solvent, such as heating the dispersion under reduced pressures, the solvent is primarily removed first, allowing all or most of the water to remain in the composition.

Suitable water-miscible volatile solvents for vesicle dispersion preparation include organic solvents such as alcohols, ethers, glycols, esters, acids, halogenated hydrocarbons, diols. The organic solvents should be miscible with water at the proportion and lower in order to effectively disperse silicones and maintain stable and uniform dispersion overtime. For the purpose of illustration, water-miscible alcohols include method, ethanol, propanol, isopropanol, butanol, and higher hydrocarbon alcohols; ethers include gylcol ethers, methylethyl ether, methyl isobutyl ether (MIBK), etc; glycols include propylene glycols, esters include esters of triglycerol, the esterification products of acid and alcohol; halogenated hydrocarbons include chloroform. Typically water-miscible organic solvents are solvents with relatively low boiling points (<100°C) or high evaporation rate, so they may be removed under vacuum with ease. The most preferred water-miscible organic solvents for this invention are volatile alcohols including methanol, ethanol, isopropanol, and propanol. These alcohols can be removed from aqueous mixtures containing silicone vesicle dispersions via vacuum stripping at ambient temperature.

The order of mixing components A), B), and C) is not critical, but typically A) and B) are first mixed and then water added to the mixture. There are no special requirements or conditions needed for effecting the mixing of components A), B), and C). The mixing can be conducted in a batch, semi-continuous, or continuous process.

The amount of components A), B), and C) can vary in the process, but typically range as follows;
A) 2 to 50 wt%, alternatively 2 to 25 wt %, or alternatively 2 to 15 wt%,
B) 2 to 50 wt%, alternatively 2 to 25 wt %, or alternatively 2 to 15 wt%,
C) sufficient amount to provide the sum of the wt% of A), B), and C) to equal 100%
The amount of B) water-miscible volatile solvent used to disperse the organopolysiloxane depends on the type of organopolysiloxane and how much hydrophilic groups are present. Typically, the aqueous mixture to effective disperse silicones comprises of 5 to 80 parts of solvent and 20 to 95 parts of water; alternatively 5 to 50 parts of water, or alternatively 10 to 40 parts water.

Step II in the process of the present invention is mixing the aqueous dispersion formed in Step I to form vesicles. There are no special requirements or conditions needed to effect the mixing and formation of vesicles. Mixing techniques can be simple stirring, homogenizing, sonalating, and other mixing techniques known in the art to effect the formation of vesicles in aqueous dispersions. The mixing can be conducted in a batch, semi-continuous, or continuous process.

The formation of vesicles can be confirmed by techniques common in the state of the art. Typically, vesicles have a lamellar phase structure which exhibit birefringence when examined with a cross polarizing microscope. Alternatively, the formation of vesicles can be demonstrated by Cyro-Transmission Electron Microscopy (Cryo-TEM) techniques. Particle size measurements can also be used to indicate that the organopolysiloxanes are sufficiently dispersed in aqueous medium typical of vesicle sizes. For example, average particle sizes of less than 0.500 µm (micrometers), are typical for dispersed vesicles. Vesicles having an average particle size of less than 0.200 µm, or 0.100 µm are possible with the teachings of the present invention.

Step III in the process of the present invention is optional, and involves removing the water miscible volatile solvent, component B). Typically, the water miscible volatile solvent is removed by known techniques in the art, such as subjecting the vesicle composition to reduced pressures, while optionally heating the composition. Devices illustrative of such techniques include rotary evaporators and thin film strippers.

The present invention also relates to the vesicle compositions produced by the methods, as described supra. The formation of vesicles can be confirmed by techniques common in the state of the art. Typically, vesicles having a lamellar phase structure which exhibit birefringence when examined with a cross polarizing microscope. Alternatively, the formation of vesicles can be demonstrated by Cyro-Transmission Electron Microscopy (Cryo-TEM) techniques. Particle size measurements can also be used to indicate that the organopolysiloxanes are sufficiently dispersed in aqueous medium typical of vesicle sizes For example, average particle sizes of less than 0.500 µm (micrometers), are typical for dispersed vesicles. Vesicles having a average particle size of less than 0.200 µm, or 0.100 µm are possible with the method of the present invention.

The present invention further relates to vesicle compositions further comprising a personal, household, or health care ingredient. Thus, the vesicle compositions can be used to entrap, and subsequently deliver after application, a personal, household care, or health care ingredient. A listing of possible personal, household, or health care ingredients is taught in WO 03/101412, which is incorporated herein by reference. The personal or health care ingredient can also be selected from a personal or health care "active", that is, any compound known to have either cosmetic and/or pharmaceutical activity. A representative listing of such personal or health care actives are disclosed in US Patent 6,168,782, which is hereby incorporated by reference.

Compositions prepared according to the invention can be used in various over-the-counter (OTC) personal care compositions, health care compositions, and household care compositions, but especially in the personal care arena. Thus, they can be used in antiperspirants, deodorants, skin creams, skin care lotions, moisturizers, facial treatments such as acne or wrinkle removers, personal and facial cleansers, bath oils, perfumes, colognes, sachets, sunscreens, pre-shave and after-shave lotions, liquid soaps, shaving soaps, shaving lathers, hair shampoos, hair conditioners, hair sprays, mousses, permanents, depilatories, hair cuticle coats, make-ups, color cosmetics, foundations, blushes, lipsticks, lip balms, eyeliners, mascaras, oil removers, color cosmetic removers, nail polishes, and powders.

### EXAMPLES

The following examples are presented to further illustrate the compositions and methods of this invention, but are not to be construed as limiting the invention. All parts and percentages in the examples are on a weight basis and all measurements were obtained at about 23°C, unless indicated to the contrary.

The vesicle compositions were analyzed via Cyro-TEM techniques according to the following procedure. Around 2.3 µl of aqueous sample solution was loaded using a micropipette on a lacey carbon film coated Cu TEM grid that was cleaned and rinsed with acetone and chloroform. The samples were diluted to about 5% solution with de-ionized water. The excess fluid on the grid surface was removed by blotting the surface with a filter paper for 1.5 second to make an aqueous thin film for TEM. The grid was then plunged into a liquid ethane contained in a small vessel located in a larger liquid nitrogen vessel under -175 °C atmosphere in the cryo-plunge system to vitrify the water film on the grid and to avoid water crystallization. The quenched sample grid was transferred in to the cryo-grid box in the cryo-plunge system. The grid box containing the sample was transferred into a Gatan cryo-transfer system filled with liquid nitrogen and loaded in a cryo-TEM stage, which has been positioned in the cryo-transfer system and cooled down to below -160 °C. The sample was loaded in TEM (JEOL 2000FX) and the images were observed at below -160 °C. A much colder finger, cooled to -180 °C in TEM using liquid nitrogen, was present to reduce any possible contamination on the cold specimen surface under high vaccum during TEM analysis. The digital images, as shown herein, were taken using a Gatan CCD camera attached at the bottom of the TEM column and Digital Micrograph software.

### Example 1 (Reference example)

### Silicone polyethers used for preparation of vesicle dispersions

A series of silicone polyethers were prepared, as illustrated in Table 1 below. This series were "rake type" silicone polyethers since they were derived from various dimethyl-methylhydrogen-polysiloxanes of the general formula, MDₓD'yM and allyloxy polyethers.
The MDₓD'yM siloxanes were prepared according to known techniques. The silicone polyethers were prepared using well know platinum catalyzed hydrosilylation techniques, such as those disclosed in US Patent 2,823,218.

The D/D' ratio corresponds to x / y ratio in the formula MDₓD'yM and total DP is the sum of x and y. EO7 is allyoxyl poly(ethylene oxide) having about seven EO repeat units, and EO12 is allyoxyl poly(ethylene oxide) having about 12 EO repeat units; both were obtained from Dow Chemical Company (Midland, MI).

**Table 1**

| **Siloxane structure** | **MID₂₇D'₃M** | **MD₂₂D'₂M** | **MD_{28.8}D'_{1.22}M** | **MD_{46.88}D'_{3.12}M** | **MD₇₀D'₃M** |
|---|---|---|---|---|---|
| **D / D' ratio** | **9.0** | **10** | **15** | **15** | **23.3** |
| **Total dp, D+D'** | **30** | **24** | **30** | **50** | **73** |
| **EO7 polyether** | | **SPE 1** | **SPE 2** | **SPE 3** | |
| **EO12 polyether** | **SPE 4** | **SPE 5** | **SPE 6** | **SPE 7** | **SPE 8** |

For MDₓD'_{y}M rake-type SPEs, D/D' ratio represents the relative ratio of the number of siloxane repeat units to the number of polyether substituted units on the siloxane polyethers, and a direct measure of hydrophobic to hydrophilic ratio of the SPEs. The lower the D/D' ratio, the more hydrophilic the SPEs, and more readily dispersible the SPE is in water.

### Example 2

### Effect of D/D' ratio of SPEs and alcohol on vesicle dispersion preparation

SPEs were generally found to spontaneously disperse in water, forming vesicles, when the D/D' was 12 or lower, as summarized in Table 2 below. When the D/D' ratio of the SPE was 15 or higher, the SPEs were less dispersible in water, and the rate of SPE dispersion in water could be very slow to incomplete. This example demonstrates the time needed to disperse SPE 6 (from Example 1) in water and how much faster it disperses when a small amount of isopropanol was present.

When D/D' was greater than 15, the SPEs did not spontaneously disperse in water. For example, the SPEs derived from MD₇₀D'₃M (such as SPE 8) were not dispersible at all in water, even over more than a period of 2 months.

Examples 2A and 2 B show how SPE 6, a silicone polyether of MD_{28.8}D'_{1.22}M siloxane structure dispersed in water alone and in the presence of 5 % isopropanol, respectively. It took about 3 weeks of continuous mixing to form a homogeneous dispersion in water as opposed to less than 1 day when 5 % by weight of isopropanol present in water.

Homogeneous dispersions of SPE 3 and SPE 7, silicone polyethers of MD_{46.88}D'_{3.12}M siloxane backbone with D/D' of 15, were also made into homogeneous dispersions within 2 days with aid of 5 % isopropanol, as shown in Examples 2C and 2D.

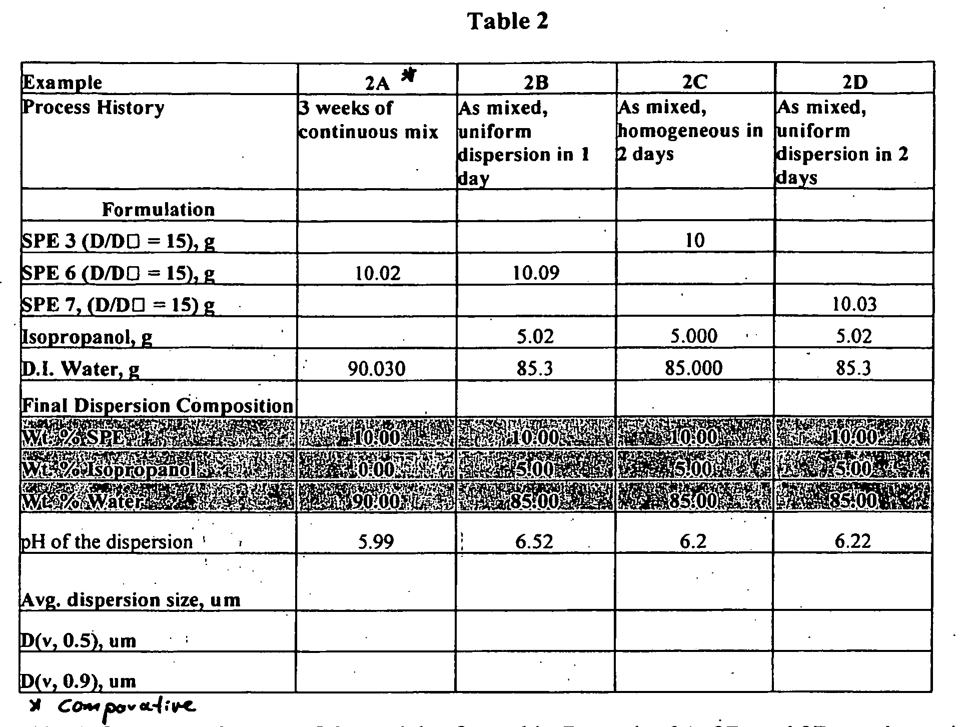

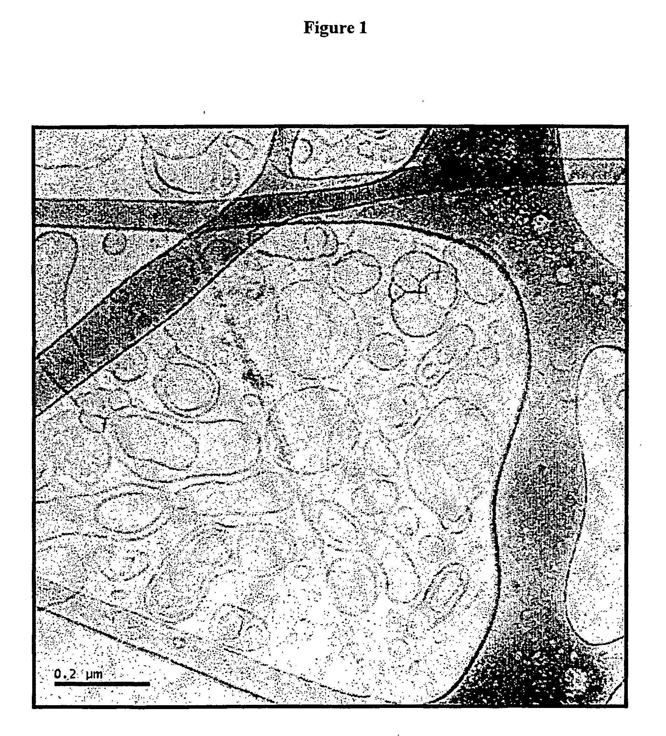

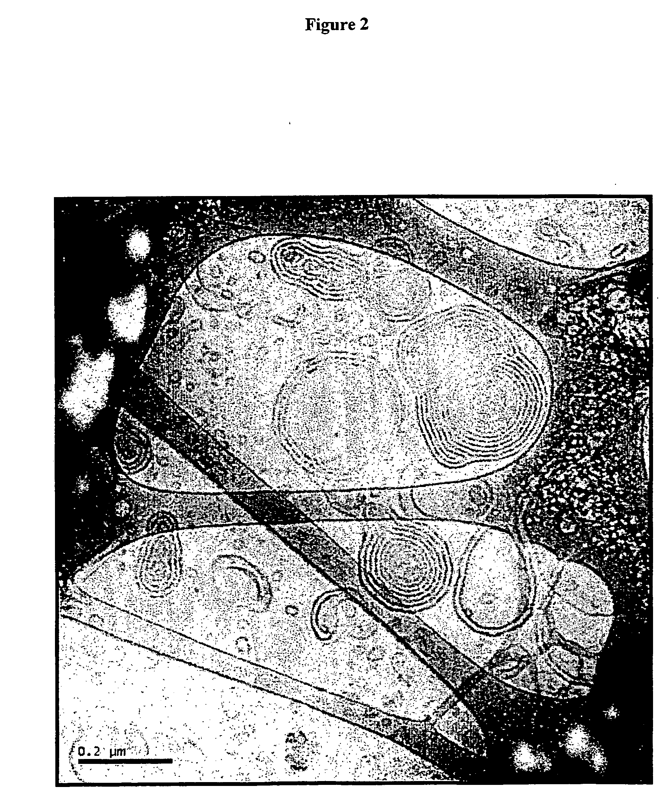

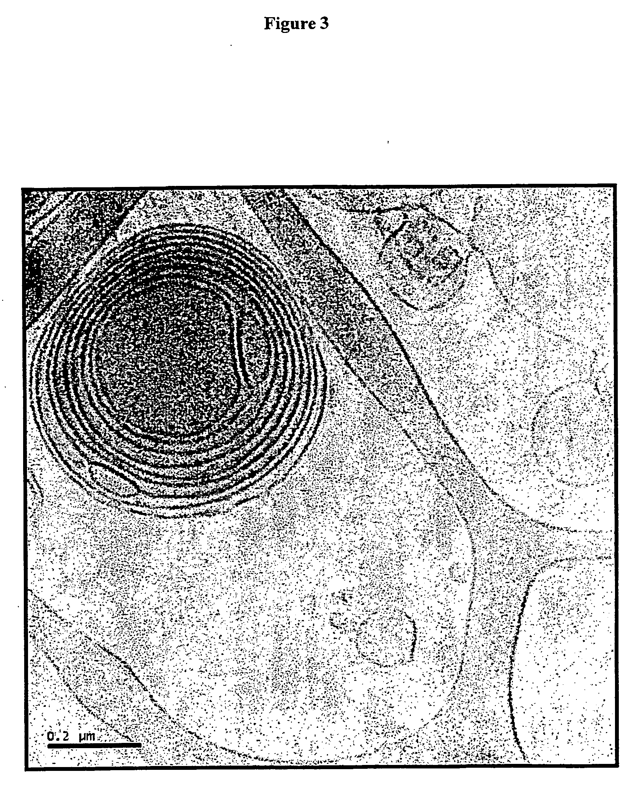

Cryo-TEM images of the vesicles formed in Examples 2A, 2B, and 2D are shown in figures 1 - 3 respectively. These images show the presence of single lamellar vesicles in Example 2A, and presence of multi-lamellar vesicles in Examples 2B and 2D.

### Example 3

### Comparative example

A dispersion of SPE 8 in water was attempted by incorporating 10.0 g of SPE 8 into 90.0 g of de-ionized water in a glass jar. The jar was subjected to mixing on a rotowheel mixer. Even after 4 weeks of mixing the SPE remained as separated in water, no dispersion was formed.

### Example 4

### Preparation of 20% and 30% SPE 8 Vesicle Dispersions

### Example A4: Vesicle dispersion of 14.4% SPE 8 dispersion in 28.7% isopropanol and 56.9 % water.

A dispersion was prepared by first adding SPE 8 into isopropanol with continuous mixing. Then, water was gradually added with continuous stirring. The resulting final mixture was clear, and a homogenous dispersion. SPE vesicles of 31 nm average sizes were observed and the dispersion has a clear water-like appearance due to the size of the vesicles.

### Example 4B: Vesicle dispersion of 20.0% SPE 8 in 80. 0 % water.

A homogeneous dispersion of SPE 8 in isopropanol and water was prepared, following a similar procedure for Example 4A. This dispersion was further stripped under vacuum at ambient temperature to remove volatile isopropanol using a Rotovapor. The theoretical amount of 74.93 g of IPA was completely removed and yielded a theoretically alcohol-free aqueous dispersion of SPE 8 vesicles. The resultant product was a milky-white, homogeneous dispersion with an average particle size of 212 nm, as measured by Nanotrac particle analyzer.

### Example 4C: vesicle dispersion of 21.2% SPE 8 in 28.8% isopropanol and 50% water.

A homogeneous vesicular dispersion of 21 nm average particle size in isopropanol and water was prepared, following a procedure similar to that of Example 4A.

### Example 4D: vesicle dispersion of 29.5 % SPE 8 in 1.0% isopropanol and 69.5% water

A homogeneous vesicular dispersion of 436 nm average particle size in water was prepared, following a procedure similar to that of Example 4B. The final mixture was a milky-white, homogenous dispersion.

| **Example #** | **4A** | **4B** | **4C** | **4D** |
|---|---|---|---|---|
| Process History | As mixed | Mixed, then stripped | As mixed | Mixed, then stripped |
| **SH 3775M SPE, g** | 43.11 | 37.59 | 64.12 | 55.89 |
| **IPA, g** | 86.23 | 74.93 | 87.00 | 75.93 |
| **D.I. Water, g** | 170.724 | 148.55 | 151.268 | 131.82 |
| Batch size, as mixed | 300 | 261.07 | 302.4 | 263.64 |
| **IPA Removed, g** | | -75.70 | | -74.10 |
| **Final batch, after strip** | | 185.37 | | 189.53 |
| **Wt. % Polymer** | **14.4** | **20.0** | **21.2** | **29.5** |
| **Wt. % IP A** | **28.7** | **0.0** | **28.8** | **1.0** |
| **Wt. % Water** | **56.9** | **80.0** | **50.0** | **69.5** |
| **Appearance of dispersion** | Clear water-like liquid | Milky white material | Clear water-like liquid | Milky white material |
| **Avg. particle size, µm** | **0.031** | **0.212** | **0.021** | **0.436** |
| **D(v,0.5), µm** | 0.029 | 0.178 | 0.019 | 0.353 |
| **D(v, 0.9), µm** | 0.044 | 0.428 | 0.036 | 0.914 |

### Example 5

### Effect of isopropanol concentration on vesicle dispersion size

The ease of dispersing silicone polyethers and, in some cases, the size and structure of the vesicles in the final water dispersions depends on the amount of isopropanol. This example illustrates this effect with two rake-type SPEs, SPE 7 and 8 as identified in Example 1.

### Examples 5A, 5B, and 5C

Dispersions of SPE 8 in water containing 5% to 20 wt.% isopropanol were prepared according to the procedure described previously, according to the formulations summarized in Table 5. Example 5C was prepared by subjecting the Example 5B dispersion to further vaccum stripping to remove most of the IPA.

### Example 5D and 5E

A homogeneous vesicle dispersion of SPE 7 was made in 20 % IPA and 70 % water having an average dispersion size of only 77 nm, according to the previously described procedure, as summarized as Example 5D. The isopropanol was the removed by vacuum stripping, resulting in a dispersion (Example 5E) having an average dispersion size of only 49 nm in water, as summarized in Table 5.

**Table 5**

| **Example #** | **5A** | **5B** | **5C** | **5D** | **5E** |
|---|---|---|---|---|---|
| **Process History** | **As mixed** | **As mixed** | **Mixed, then stripped** | **As mixed** | **Mixed, then stripped** |
| **SH 3775M SPE, g** | 10.025 | 30.012 | 20 | | |
| **18841-91 SPE, g** | | | | 30.006 | 20.0 |
| **IPA, g** | 10.005 | 60.082 | 10 | 60.172 | 40.0 |
| **D.I. Water, g** | 80.044 | 210.063 | 140 | 210.757 | 140.0 |
| **Final Dispersion Composition** | | | | | (-40 g alcohol) |
| **Wt. % Polymer** | **10.02** | **10.00** | **11.76** | **9.97** | **12.50** |
| **Wt. % IPA** | **10.00** | **20.02** | **5.88** | **20.00** | **0.00** |
| **Wt. % Water** | **79.98** | **69.98** | **82.35** | **70.03** | **87.50** |
| pH of the dispersion | 5.87 | 7.14 | 6.81 | 6.35 | 6.38 |
| **Avg. dispersion size, um** | **1.427** | **0.106** | **0.099** | **0.077** | **0.049** |
| **D(v, 0.5), um** | 0.955 | 0.092 | 0.091 | 0.071 | 0.072 |
| **D(v, 0.9), um** | 3.789 | 0.178 | 0.156 | 0.112 | 0.104 |

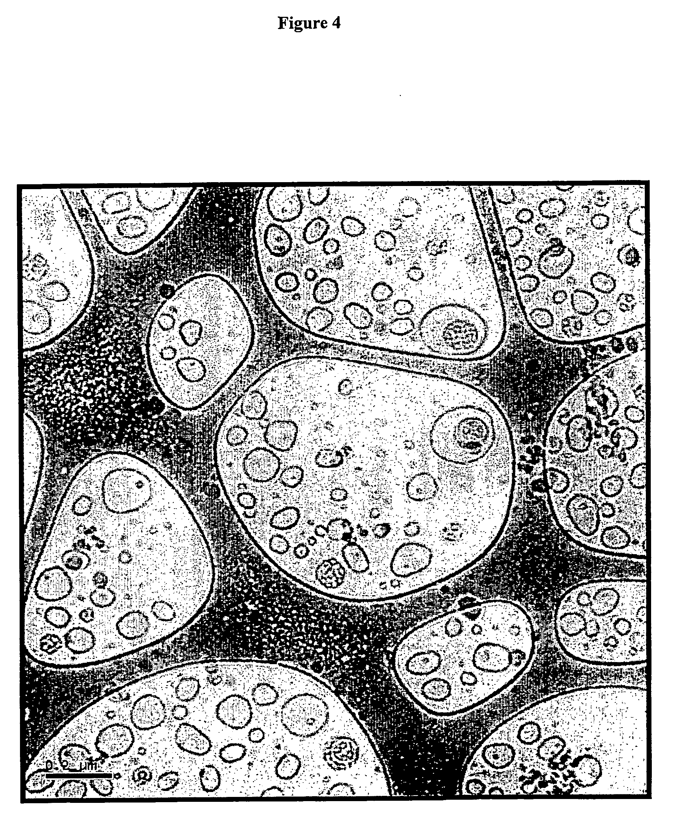

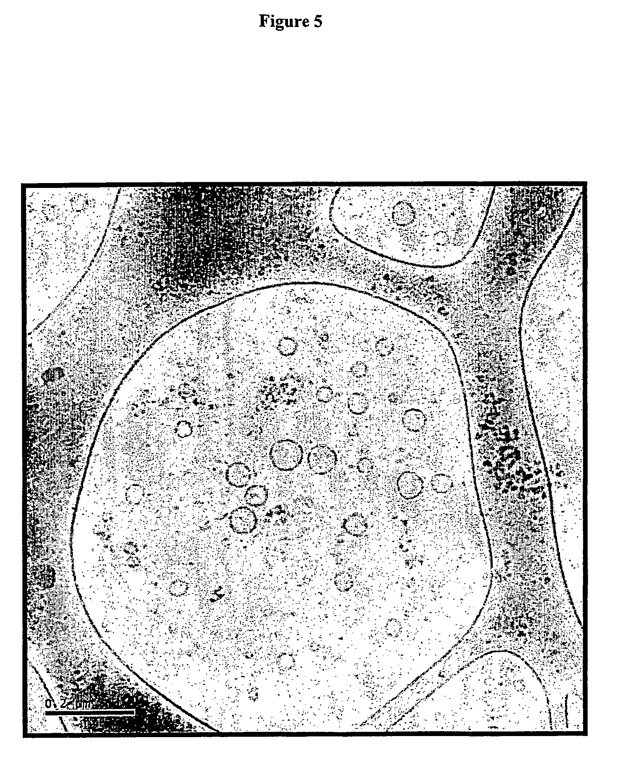

Cryo-TEM images confirmed the silicone polyether dispersions were nano-scale vesicles and aggregates of vesicles. The actual sizes observed were consistent with the particle size measured by Nanotrac particle analyzer. The cryo-TEM images of SPE 8 dispersion (Example 5C) and that of SPE 7 dispersion (Example 5E) are shown in figures 4 and 5 respectively.

### Example 6

### SPE vesicle dispersions containing Vitamin A palmitate

The method of this invention can also be used to entrap and retain oil-soluble substances into vesicles in aqueous dispersions. This is very desirable and beneficial utility of vesicles and vesicular aggregates prepared from silicone polyethers. As many personal care actives & ingredients are not water-soluble or not water-compatible. This makes it not practical to incorporate such ingredients as vitamins, fragrances, sunscreens, and oils directly into water-based or water-continuous end-product formulations, such as creams, lotions, gels, etc.

This series of examples shows that vitamin A palmitate (a preferred form of vitamin A for skin care products) can be successfully incorporated into SPE based vesicles in water dispersions, using the method of this invention. Results and formulations are summarized in Table 6.

### Examples 6A and 6B

### Vitamin A plamitate entrapped SPE vesicles in partially and fully stripped aqueous dispersions.

Vitamin A palmitate (VAP) was first dispersed in isopropanol at 50/50 ratio by weight to keep VAP in a liquid form for ease of mixing. The VAP/IPA was then mixed in with SPE 8, followed by incorporation of isopropanol to form a homogeneous mixture. While under continuous mixing, water was gradually and continuously incorporated into the mixture containing the SPE, VAP and IPA. Then, the mixture was homogenized, using an APV benchtop homogenizer, to further narrow and reduce the dispersion size. The homogenized dispersion was subsequently stripped to remove isopropanol under vaccum at ambient temperature, using a Rotovapor.

The 6A example was a partially stripped dispersion having about 10% IPA remaining and an average dispersion size of 0.229 um. The 6B example was a fully stripped dispersion containing 20.8% SPE, 4.5% VAP, 2.2% IPA and 72.5% water. Example 6B was a homogeneous, beige milky dispersion having an average vesicle size of 0.974 um.

### Example 6C

### Vitamin A palmitate loaded dispersion made without homogenization using ethano.

This dispersion was prepared by first dispersing vitamin A palmitate into SPE 8 in the presence of a small quantity of ethanol. The prescribed amount of ethanol was mixed in with SPE / VAP to form a homogeneous mixture. While under continuous mixing, water was gradually and continuously incorporated into the mixture. A homogeneous mixture was obtained. The mixture was then subjected to vaccum stripping to remove the volatile alcohol. The final dispersion had a composition of 4.5% VAP, 20.7% SPE, 2.9% EtOH and 72.0 % water. The dispersion had an average dispersion size of 0.763 um.

### Examples 6D and 6E

### Vitamin A palmitate loaded dispersions made with EtOH.

Ethanol was used as dispersing aid to prepare these VAP loaded SPE vesicles in water dispersions. The procedures followed the ones that used to make Example 6A and 6B, except EtOH used in palce of IPA. The final dispersion compositions are shown in Table 6. The two dispersions had an average dispersion size of 0.284 um and 0.331 um, respectively.

**Table 6**

| **Example #** | **6A** | **6B** | **6C** | **6D** | **6E** |
|---|---|---|---|---|---|
| Alcohol type used | IPA | IPA | EtOH | EtOH | EtOH |
| Process History | Mixed, Homog., Partially Stripped | Mixed, Homog., Fully Stripped | Mixezd Stripped | Mixed, Homog., Partially Stripped | Mixed, Homog, Fully Stripped |
| **VAP (vitamin A palmitate), g** | 3.96 | 3.91 | 9.89 | 3.94 | 3.69 |
| **Alcohol, g** | 32.71 | 32.32 | 81.61 | 32.54 | 30.50 |
| **SPE 8, g** | 18.35 | 18.13 | 45.73 | 18.23 | 17.09 |
| **Water, g** | 63.96 | 63.19 | 159.01 | 63.40 | 59.42 |
| **Batch size, as mixed, g** | 118.97 | 117.55 | 296.23 | 118.11 | 110.70 |
| **Alcohol Removed, g** | 23.48 | 30.37 | 75.30 | 22.21 | 28.47 |
| **Batch size, after strip, g** | 95.49 | 87.18 | 220.93 | 95.90 | 82.23 |
| **Final Dispersion composition** | | | | | |
| **Wt, % VAP** | **4.1** | **4.5** | **4.5** | **4.1** | **4.5** |
| **Wt. % Alcohol** | **9.7** | **2.2** | **2.9** | **10.8** | **2.5** |
| **Wt. % SPE Polymer** | **19.2** | **20.8** | **20.7** | **19.0** | **20.8** |
| **Wt. % Water** | **67.0** | **72.5** | **72.0** | **66.1** | **72.3** |
| **Appearance of Dispersion** | Beige milky dispersion | Beige milky dispersion | Beige milky dispersion; small oil droplets atop | Beige milky dispersion | Beige milky dispersion |
| **Average particle size, um** | **0.2291** | **0.974** | **0.763** | **0.2842** | **0.331** |
| **D(v, 0.5), um** | 0.1602 | 0.995 | 0.374 | 0.1624 | 0.2103 |
| **D(v, 0.9), um** | 0.513 | 1.783 | 1.987 | 0.727 | 0.785 |

## Claims

1. A process for preparing a vesicle composition comprising;
I) combining
A) an organopolysiloxane having at least one hydrophilic substituent group,
B) a water miscible volatile solvent, with water to form an aqueous dispersion,
II) mixing the aqueous dispersion to form vesicles, wherein the organopolysiloxane is a silicone polyether having a structure represented by: 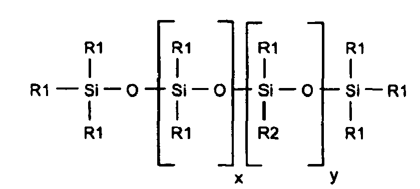 or 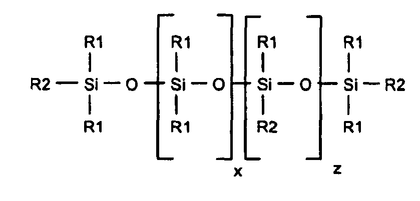 or 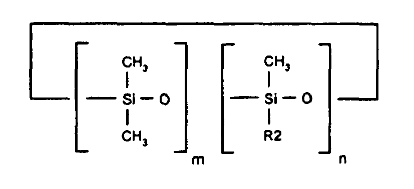
where R1 represents an alkyl group containing 1-6 carbon atoms;
R2 represents the group -(CH₂)ₐO(C₂H₄O)_{b}(C₃H₆O)_{c}R3;
x is 1-1,000; y is 1-500; z is 1-500; m has a value of 3-5; n is one; a is 3-6;
b is 4-20; c is 0-5; x/y and x/z ranges from 5/1 to 50/1;
and R3 is hydrogen, a methyl group, or an acyl group.

2. The process of claim 1 wherein the aqueous dispersion contains;
A) 5 to 50 wt % of the organopolysiloxane,
B) 5 to 50 wt% of the water miscible solvent,
C) sufficient amount of water to provide the sum of the wt% of A), B), and C) to equal 100 wt %.

3. The process of claim 1 wherein the silicone polyether has the structure, 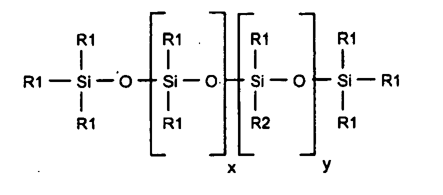 x is from 1 to 1000, y is from 1 to 500, R1 is methyl,
R2 represents the group -(CH₂)ₐO(C₂H₄O)_{b}(C₃H₆O)_{c}R3 where a is 3-6; b is 4-20; c is 0-5,and R3 is hydrogen, a methyl group, or an acyl group.

4. The process of claim 1 wherein the water miscible volatile solvent is an alcohol.

5. The process of claim 1 wherein the water miscible volatile solvent is isopropyl alcohol.

6. The process of claim 1, further comprising removing the water miscible volatile solvent from the vesicles.

7. The product resulting from the process of any one of claims 1 - 5.

8. A personal, household, or health care product comprising the product of claim 7.

## Patentansprüche

1. Verfahren zur Herstellung einer Vesikelzusammensetzung umfassend:
I) Kombinieren von
A) einem Organopolysiloxan mit wenigstens einer hyrophilen Substituentengruppe,
B) einem wassermischbaren flüchtigen Lösungsmittel mit Wasser, um eine wässrige Dispersion zu bilden,
II) Mischen der wässrigen Dispersion, um Vesikel zu bilden,
worin das Organopolysiloxan ein Siliconpolyether mit einer Struktur, wiedergegeben durch: 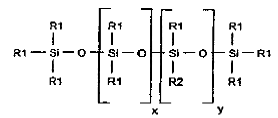 oder 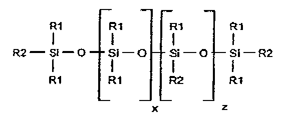 oder 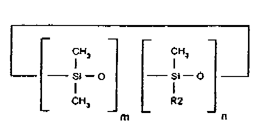 ist, worin R1 für eine Alkylgruppe mit 1 bis 6 Kohlenstoffatomen steht, R2 für die Gruppe -(CH₂)ₐO(C₂H₄O)_{b}(C₃H₆O)_{c}R3 steht, x gleich 1 bis 1.000 ist, y gleich 1 bis 500 ist, z gleich 1 bis 500 ist, m einen Wert von 3 bis 5 aufweist, n gleich 1 ist, a gleich 3 bis 6 ist, b gleich 4 bis 20 ist, c gleich 0 bis 5 ist, x/y und x/z im Bereich von 5/1 bis 50/1 liegen, und R3 Wasserstoff, eine Methylgruppe oder eine Acylgruppe ist.

2. Verfahren nach Anspruch 1, wobei die wässrige Dispersion enthält:
A) 5 bis 50 Gew.% des Organopolysiloxans,
B) 5 bis 50 Gew.-% des wassermischbaren Lösungsmittels,
C) eine ausreichende Menge an Wasser, um eine Summe der Gewichtsprozentanteile von A), B) und C) von gleich 100 Gew.-% bereitzustellen.

3. Verfahren nach Anspruch 1, worin der Siliconpolyether die Struktur 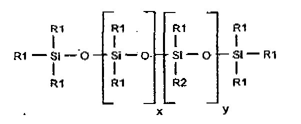 aufweist,
x gleich 1 bis 1.000 ist, y gleich 1 bis 500 ist, R1 Methyl ist, R2 für die Gruppe -(CH₂)ₐO(C₂H₄O)_{b}(C₃H₆O)_{c}R3 steht, worin a gleich 3 bis 6 ist, b gleich 4 bis 20 ist, c gleich 0 bis 5 ist und R3 Wasserstoff, eine Methylgruppe oder eine Acylgruppe ist.

4. Verfahren nach Anspruch 1, worin das wassermischbare flüchtige Lösungsmittel ein Alkohol ist.

5. Verfahren nach Anspruch 1, worin das wassermischbare flüchtige Lösungsmittel Isoproylalkohol ist.

6. Verfahren nach Anspruch 1, das zusätzlich Entfernen des wassermischbaren flüchtigen Lösungsmittels aus den Vesikeln umfasst.

7. Produkt, resultierend aus dem Verfahren nach einem der Ansprüche 1 bis 5.

8. Körperpflege-, Haushalts- oder Gesundheitsfürsorgeprodukt, enthaltend das Produkt nach Anspruch 7.

## Revendications

1. Procédé de préparation d'une composition de vésicule comprenant :
I) la combinaison
A) d'un organopolysiloxane présentant au moins un groupe substituant hydrophile,
B) d'un solvant volatil miscible à l'eau, avec de l'eau pour former une dispersion aqueuse,
II) le mélange de la dispersion aqueuse pour former des vésicules, dans lequel l'organopolysiloxane est un silicone polyéther présentant une structure représentée par : 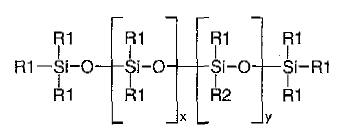
ou 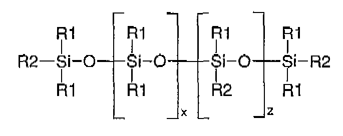 ou 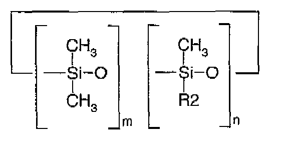 dans laquelle R1 représente un groupe alkyle ayant 1-6 atomes de carbone ;
R2 représente le groupe -(CH₂)ₐO(C₂H₄O)_{b}(C₃H₆O)_{c}R3 ;
x est égal à 1-1000 ; y est égal à 1-500 ; z est égal à 1-500 ; m présente une valeur de 3-5 ; n est égal à 1 ; a est égal à 3-6 ;
b est égal à 4-20 ; c est égal à 0-5 ; x/y et x/z sont compris entre 5/1 et 50/1 ;
et R3 est un atome d'hydrogène, un groupe méthyle ou un groupe acyle.

2. Procédé selon la revendication 1, dans lequel la dispersion aqueuse contient :
A) de 5 à 50 % en poids de l'organopolysiloxane,
B) de 5 à 50 % en poids du solvant miscible à l'eau,
C) une quantité suffisante d'eau pour obtenir la somme des % en poids de A), B) et C) égale à 100 % en poids.

3. Procédé selon la revendication 1, dans lequel le silicone polyéther présente la structure, 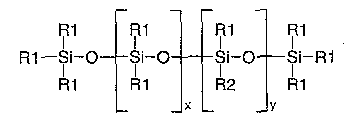 x est compris entre 1 et 1000, y est compris entre 1 et 500, R1 est le groupe méthyle,
R2 représente le groupe -(CH₂)ₐO(C₂H₄O)_{b}(C₃H₆O)_{c}R3
dans lequel a est égal à 3-6 ; b est égal à 4-20 ; c est égal à 0-5 et R3 est un atome d'hydrogène, un groupe méthyle ou un groupe acyle.

4. Procédé selon la revendication 1, dans lequel le solvant volatil miscible à l'eau est un alcool.

5. Procédé selon la revendication 1, dans lequel le solvant volatil miscible à l'eau est l'alcool isopropylique.

6. Procédé selon la revendication 1 comprenant de plus l'élimination du solvant volatil miscible à l'eau des vésicules.

7. Produit résultant du procédé selon l'une quelconque des revendications 1-5.

8. Produit pour la personne, la maison ou la santé comprenant le produit de la revendication 7.
